# EUROPÄISCHE PATENTSCHRIFT

(11) **EP 3 130 306 B1**
(45) Veröffentlichungstag und Bekanntmachung des Hinweises auf die Patenterteilung: **10.10.2018**
(21) Anmeldenummer: 15180669.2
(22) Anmeldetag: 12.08.2015
(51) Int. Cl.: A61C 3/03, A61C 1/05

(54) **DENTALES ODER DENTALCHIRURGISCHES ULTRASCHALLWERKZEUG**
DENTAL OR DENTAL-SURGICAL ULTRASONIC TOOL
OUTIL ULTRASONIQUE DENTAIRE OU DE CHIRURGIE DENTAIRE

(43) Veröffentlichungstag der Anmeldung: 15.02.2017
(73) Patentinhaber: W & H Dentalwerk Bürmoos GmbH, 5111 Bürmoos (AT)
(72) Erfinder: ANGERMEIER, Bernd, 5112 Lamprechtshausen (AT); BRUGGER, Dr., Wilhelm, 5071 Wals-Siezenheim (AT)
(74) Vertreter: Benda, Ralf

(56) Entgegenhaltungen:
- EP-A1- 1 974 680
- WO-A1-2008/038307
- US-A- 4 021 920
- US-A- 5 022 857
- US-A- 5 575 650
- US-A1- 2010 178 631
- US-A1- 2011 143 308
- US-A1- 2011 229 845

## Beschreibung

Die vorliegende Erfindung betrifft ein dentales oder dentalchirurgisches Ultraschallwerkzeug mit einem länglichen Werkzeugkörper, einer Anschlussvorrichtung zum lösbaren Anschluss an eine Ultraschallquelle, einer Flüssigkeitsleitung und einem Arbeitsabschnitt, der ausgebildet ist, mittels von der Ultraschallquelle übertragener Ultraschallschwingungen in einen zu behandelnden Knochen einzudringen.

Ein derartiges dentales oder dentalchirurgisches Ultraschallwerkzeug ist aus der Patentschrift US 8,109,931 B2 und der Anmeldeschrift WO 2008/038307 A1 bekannt. Zur Versorgung der Behandlungsstelle mit Kühlflüssigkeit ist an dem distalen Ende des Arbeitsabschnitts eine mit der Flüssigkeitsleitung verbundene Öffnung vorgesehen. Bei der WO'307 sind zusätzlich an der Mantelfläche parallel zur Längsachse verlaufende Längskanäle zur Entfernung von Knochenmaterial vorgesehen.

Der vorliegenden Erfindung liegt die Aufgabe zugrunde ein dentales oder dentalchirurgisches Ultraschallwerkzeug zu schaffen, dessen Verteilung der Kühlflüssigkeit, Kühlwirkung für die gesamte Spitze und dessen Handhabung verbessert ist.

Diese Aufgabe wird gemäß der vorliegenden Erfindung durch ein dentales oder dentalchirurgisches Ultraschallwerkzeug mit den Merkmalen des Anspruchs 1 gelöst. Besonders vorteilhafte Ausführungsformen sind in den Unteransprüchen angeführt.

Das dentale oder dentalchirurgische Ultraschallwerkzeug umfasst einen länglichen Werkzeugkörper, eine Anschlussvorrichtung zum lösbaren Anschluss des Ultraschallwerkzeugs an eine Ultraschallquelle, wobei die Anschlussvorrichtung an einem ersten Ende des Werkzeugkörpers vorgesehen ist, einen an einem zweiten Ende des Werkzeugkörpers vorgesehenen Arbeitsabschnitt, der sich entlang einer Längsachse erstreckt und der ausgebildet ist, aufgrund der von der Ultraschallquelle erzeugten und über die Anschlussvorrichtung und den Werkzeugkörper übertragenen Ultraschallschwingungen in einen zu behandelnden Knochen einzudringen, und eine Flüssigkeitsleitung, die sich durch den Werkzeugkörper in Richtung des Arbeitsabschnitts erstreckt und in zumindest einer Öffnung endet, die zum Austritt einer in der Flüssigkeitsleitung fließenden Flüssigkeit aus dem Ultraschallwerkzeug vorgesehen ist. Der Arbeitsabschnitt weist eine distale Stirnfläche mit mehreren Schneidkanten und eine sich von der distalen Stirnfläche in Richtung des Werkzeugkörpers erstreckende Mantelfläche auf, wobei in der Mantelfläche zumindest ein Kanal zur Flüssigkeitsleitung vorgesehen ist, der sich von der distalen Stirnfläche in Richtung des Werkzeugkörpers wendelförmig um die Längsachse des Arbeitsabschnitts erstreckt.

Gemäß einem Ausführungsbeispiel ist die zumindest eine Öffnung der Flüssigkeitsleitung an der Mantelfläche des Arbeitsabschnitts in dem zumindest einen Kanal zur Flüssigkeitsleitung oder unmittelbar anschließend an den zumindest einen Kanal zur Flüssigkeitsleitung angeordnet.

Das Vorsehen der zumindest einen Öffnung der Flüssigkeitsleitung, vorzugsweise von mehreren Öffnungen, an der Mantelfläche des Arbeitsabschnitts bewirkt eine bessere Verteilung der Kühlflüssigkeit auf dem gesamten Arbeitsabschnitt und damit eine stärkere Kühlwirkung, insbesondere wenn der Arbeitsabschnitt bereits in den Knochen eingedrungen ist und somit nicht nur an der distalen Stirnfläche, sondern auch an der seitlichen Mantelfläche des Arbeitsabschnitts eine Kühlung vorteilhaft ist. Durch die Anordnung der zumindest einen Öffnung der Flüssigkeitsleitung, vorzugsweise von mehreren Öffnungen, in dem oder unmittelbar anschließend an den zumindest einen Kanal zur Flüssigkeitsleitung wird sichergestellt, dass die Flüssigkeit zur distalen Stirnfläche und damit auch an die Behandlungsstelle gelangt, so dass auch diese beiden Bereiche ausreichend mit Flüssigkeit versorgt und gekühlt werden.

Der zumindest eine Kanal zur Flüssigkeitsleitung ist wendelförmig um die Längsachse des Arbeitsabschnitts ausgebildet, so wie dies im Folgenden im Detail beschrieben ist.

Durch die wendelförmige Ausbildung des zumindest einen Kanals zur Flüssigkeitsleitung wird insbesondere eine verbesserte Ausformung der Kavität im Knochen und eine erleichterte Handhabung des Ultraschallwerkzeugs erreicht: Da das Ultraschallwerkzeug während des Betriebs primär entlang der Längsachse des Arbeitsabschnitts vorwärts und zurück vibriert, entsteht über den gesamten Umfang des Knochens betrachtet durch das Vorsehen von zumindest einem wendelförmigen Kanal ein glatterer oder homogenerer Schnitt im Knochen (an der Seitenwand der Kavität) im Vergleich zu einem Kanal, der parallel zur Längsachse des Arbeitsabschnitts verläuft. Um eine gleichmäßige Formung der durch das Ultraschallwerkzeug in dem Knochen gebildeten Kavität zu erreichen, dreht der Anwender während des Vordringens des Ultraschallwerkzeugs in den Knochen das Ultraschallwerkzeug um die Längsachse des Arbeitsabschnitts. Dieses Drehen durch den Anwender wird ebenfalls erleichtert (es ist weniger "holprig"), da der Schnitt im Knochen durch den zumindest einen wendelförmigen Kanal glatter oder homogener ist.

Das dentale oder dentalchirurgische Ultraschallwerkzeug ist vorzugsweise aus Metall gefertigt, insbesondere aus Stahl.

Der längliche Werkzeugkörper ist vorzugsweise zumindest an einem Abschnitt oder über seine gesamte Länge zylindrisch ausgebildet. Der längliche Werkzeugkörper weist eine Biegung auf, so dass der Werkzeugkörper insbesondere zwei gewinkelt zueinander angeordnete Abschnitt aufweist. Der Winkel dieser beiden Abschnitte zueinander beträgt zum Beispiel zwischen 5° - 90°, bevorzugt zwischen 10° - 75°.

Die Anschlussvorrichtung ist zum lösbaren Anschluss des Ultraschallwerkzeugs an eine Ultraschallquelle und insbesondere auch zum lösbaren Anschluss an eine Flüssigkeitsquelle ausgebildet. Die Anschlussvorrichtung umfasst zum Beispiel ein rohrförmiges Element mit einer Innenbohrung und einem an der Wand der Innenbohrung vorgesehenen Innengewinde. Die Anschlussvorrichtung und das Ultraschallwerkzeug sind somit auf einen in der Innenbohrung aufnehmbaren Gewindestift aufschraubbar, der mit einer Ultraschallquelle und insbesondere einer Flüssigkeitsquelle verbunden ist. Der Gewindestift ist vorzugsweise Teil eines mit einer Hand haltbaren Handstücks.

Die Anschlussvorrichtung weist bevorzugt einen größeren Außendurchmesser als der Werkzeugkörper auf und ist insbesondere über einen sich verjüngenden Abschnitt, der vorzugsweise zumindest eine Ansatzfläche für ein Werkzeug zum Aufschrauben des Ultraschallwerkzeugs auf den Gewindestift aufweist, mit dem ersten Ende des Werkzeugkörpers verbunden.

Der an einem zweiten Ende des Werkzeugkörpers vorgesehene Arbeitsabschnitt weist einen im Wesentlichen zylindrischen Außenumfang auf. Die Länge des Arbeitsabschnitts ist vorzugsweise um ein mehrfaches geringer als die Länge des Werkzeugkörpers. Die axiale Ausdehnung des Arbeitsabschnitts entlang seiner Längsachse erstreckt sich vorzugsweise von einem freien Ende des Ultraschallwerkzeugs, an dem insbesondere die distale Stirnfläche angeordnet ist, bis zumindest zu jenem Ende des zumindest einen Kanals zur Flüssigkeitsleitung, das dem anschließenden Werkzeugkörper zugewandt ist. Der Außendurchmesser des Arbeitsabschnitts ist vorzugsweise in etwa gleich groß oder größer wie der Außendurchmesser des Werkzeugkörpers. In letzterem Fall ist vorzugsweise ein sich verjüngender Übergangsabschnitt zum dem länglichen Werkzeugkörper vorgesehen, der wahlweise als Teil des Arbeitsabschnitts oder daran anschließend ausgebildet ist.

Der Arbeitsabschnitt weist eine distale Stirnfläche mit mehreren Schneidkanten und eine sich von der distalen Stirnfläche in Richtung des Werkzeugkörpers erstreckende Mantelfläche auf, wobei in der, vorzugsweise im Wesentlichen zylindrisch ausgebildeten, Mantelfläche zumindest ein Kanal zur Flüssigkeitsleitung vorgesehen ist, der sich von der distalen Stirnfläche in Richtung des Werkzeugkörpers erstreckt.

Der zumindest eine Kanal endet vorzugsweise an der distalen Stirnfläche und / oder ist mit dieser derart verbunden, dass in dem Kanal fließende Flüssigkeit auf die distale Stirnfläche und / oder auf die Behandlungsstelle übertragbar oder weiterleitbar ist und / oder Flüssigkeit von der distalen Stirnfläche und / oder der Behandlungsstelle in den zumindest ein Kanal übertreten kann.

Anschließend an den zumindest einen Kanal, insbesondere in Umfangsrichtung seitlich des Kanals, ist vorzugsweise zumindest ein Steg an dem Arbeitsabschnitt vorgesehen. Bei Vorhandensein mehrerer Kanäle auf dem Arbeitsabschnitt trennt jeweils ein Steg zwei Kanäle voneinander. Der Verlauf des zumindest eine Stegs entspricht vorzugsweise im Wesentlichen dem des zumindest einen Kanals, d.h. der zumindest eine Steg ist entweder im Wesentlichen parallel zur Längsachse des Arbeitsabschnitts oder wendelförmig um die Längsachse des Arbeitsabschnitts ausgebildet. Entsprechend endet der zumindest eine Steg vorzugsweise an der distalen Stirnfläche oder bildet mit der distalen Stirnfläche eine gemeinsame Kante. Besonders bevorzugt schließt an das freie, distale Ende des zumindest einen Stegs zumindest eine Schneidkante der distalen Stirnfläche an. Vorzugsweise ist der zumindest eine Steg erhöht gegenüber dem zumindest einen Kanal oder der zumindest eine Kanal ist als Vertiefung gegenüber dem zumindest einen Steg geformt.

Vorzugsweise erstrecken sich die Schneidkanten radial von einem Mittelpunkt der distalen Stirnfläche in Richtung der Peripherie der distalen Stirnfläche oder in Richtung der Mantelfläche. Vorzugsweise bildet jede Schneidkante einen Grat oder Kamm zweier von der Schneidkante in Richtung der distalen Stirnfläche oder des Werkzeugkörpers geneigter Flächen. Vorzugsweise kontaktiert jeder dieser geneigten Flächen an ihrer tiefsten Stelle eine weitere geneigte Fläche der nächstgelegenen Schneidkante, wodurch zwischen zwei benachbarten Schneidkanten jeweils eine Vertiefung oder eine Rinne gebildet ist. Diese Vertiefung oder Rinne ist vorzugsweise zur Leitung von Flüssigkeit vorgesehen, so wie dies im Nachstehenden noch im Detail beschrieben ist.

Vorzugsweise ist der freie oder distale Endabschnitt des zumindest einen Stegs an einer Umfangskante der distalen Stirnfläche zu einem überwiegenden Teil mit einer Schneidkante und deren geneigten Flächen verbunden. Vorzugsweise ist der zumindest eine Kanal an einer Umfangskante der distalen Stirnfläche flüssigkeitsübertragend mit der Vertiefung oder Rinne verbunden.

Die Flüssigkeitsleitung, die sich durch den Werkzeugkörper in Richtung des Arbeitsabschnitts erstreckt, ist vorzugsweise als zylindrische Bohrung in dem Werkzeugkörper ausgebildet. Die Flüssigkeitsleitung endet in zumindest einer Öffnung, die zum Austritt einer in der Flüssigkeitsleitung fließenden Flüssigkeit aus dem Ultraschallwerkzeug vorgesehen ist. Die Flüssigkeitsleitung ist mit der Anschlussvorrichtung, insbesondere mit deren Innenbohrung, verbunden oder setzt sich in der Innenbohrung der Anschlussvorrichtung fort. Falls die Flüssigkeitsleitung in mehreren Öffnungen zum Austritt einer Flüssigkeit endet, so verzweigt sich die Flüssigkeitsleitung, insbesondere im Bereich des Arbeitsabschnitts oder unmittelbar daran anschließend, vorzugsweise in mehrere Leitungsarme zur Versorgung der mehreren Öffnungen.

Die zumindest eine Öffnung, die zum Austritt einer in der Flüssigkeitsleitung fließenden Flüssigkeit aus dem Ultraschallwerkzeug vorgesehen ist, ist wahlweise an der Mantelfläche des Arbeitsabschnitts oder an der distalen Stirnfläche angeordnet. Vorzugsweise ist die zumindest eine Öffnung an der Mantelfläche in dem zumindest einen Kanal zur Flüssigkeitsleitung oder unmittelbar anschließend an den zumindest einen Kanal zur Flüssigkeitsleitung oder im Zentrum der distalen Stirnfläche angeordnet.

Vorzugsweise sind mehrere Öffnungen, zum Beispiel zwei, drei oder vier Öffnungen, zum Austritt einer in der Flüssigkeitsleitung fließenden Flüssigkeit aus dem Ultraschallwerkzeug vorgesehen. Diese Öffnungen können wahlweise alle an der Mantelfläche des Arbeitsabschnitts angeordnet sein, insbesondere in mehreren Kanälen zur Flüssigkeitsleitung oder unmittelbar anschließend an diese Kanäle. Durch das Vorsehen mehrerer Öffnungen der Flüssigkeitsleitung an der Mantelfläche des Arbeitsabschnitts wird insbesondere eine verbesserte Verteilung der Flüssigkeit an der Mantelfläche oder dem Umfang des Ultraschallwerkzeugs erreicht. Alternativ ist zumindest eine Öffnung, bevorzugt sind mehrere Öffnungen, an der Mantelfläche des Arbeitsabschnitts angeordnet, insbesondere in einem Kanal oder mehreren Kanälen zur Flüssigkeitsleitung oder unmittelbar anschließend diesen Kanal oder diese Kanäle, und es ist zumindest eine (weitere) Öffnung der Flüssigkeitsleitung an der distalen Stirnfläche vorgesehen. Damit wird eine besonderes gute Versorgung des gesamten Arbeitsabschnitts einschließlich der distalen Stirnfläche mit Flüssigkeit und eine besonders gute Kühlleistung erzielt.

Vorzugsweise sind mehrere, sich wendelförmig um die Längsachse des Arbeitsabschnitts erstreckende Kanäle und mehrere Öffnungen der Flüssigkeitsleitung vorgesehen, wobei in oder an einem Kanal jeweils nur eine Öffnung angeordnet ist.

Vorzugsweise ist zumindest in oder an einem, sich wendelförmig um die Längsachse des Arbeitsabschnitts erstreckenden Kanal keine Öffnung der Flüssigkeitsleitung vorgesehen. Insbesondere ist die Anzahl der Öffnungen der Flüssigkeitsleitung, die in oder an einem, sich wendelförmig um die Längsachse des Arbeitsabschnitts erstreckenden Kanal angeordnet sind, geringer ist als die Anzahl der (aller) Kanäle, so dass zumindest in und an einem Kanal keine Öffnung der Flüssigkeitsleitung vorgesehen ist. Dieser zumindest eine, sich wendelförmig um die Längsachse des Arbeitsabschnitts erstreckende Kanal ohne Öffnungen der Flüssigkeitsleitung ist insbesondere dazu ausgebildet ist, Flüssigkeit von der distalen Stirnfläche und / oder der Behandlungsstelle wegzuleiten.

Vorzugsweise weist der zumindest eine, sich wendelförmig um die Längsachse des Arbeitsabschnitts erstreckende Kanal eine in Richtung der distalen Stirnfläche zunehmende Tiefe auf. Die Zunahme der Tiefe in Richtung der distalen Stirnfläche beträgt (ausgehend von der geringsten Tiefe) zum Beispiel zwischen 4% und 25%, vorzugsweise zwischen 5% und 15%. Die zunehmende Tiefe des zumindest einen Kanals bewirkt ein bevorzugtes Fließen der Flüssigkeit in dem Kanal, insbesondere der aus der Öffnungen der Flüssigkeitsleitung ausgetretenen und durch den Kanal weitergeleiteten Flüssigkeit, in Richtung der distalen Stirnfläche. Vorzugsweise weist zumindest ein Kanal eine konstante Tiefe auf, insbesondere ein Kanal, der dazu ausgebildet ist, Flüssigkeit von der distalen Stirnfläche und / oder der Behandlungsstelle wegzuleiten, zum Beispiel ein im Vorstehenden genannter Kanal ohne Öffnungen der Flüssigkeitsleitung.

Vorzugsweise beträgt die Steigung des zumindest einen sich wendelförmig um die Längsachse des Arbeitsabschnitts erstreckende Kanals etwa 5 mm - 50 mm, vorzugsweise etwa 15 mm - 25 mm. Die Steigung definiert dabei die Länge des Wegs oder des wendelförmigen Kanals, der durch eine vollständige Umdrehung (um 360°) des Arbeitsabschnitts, insbesondere des wendelförmigen Kanals, zurückgelegt wird. Der kegelige Gewindeanstieg oder die Verjüngung des wendelförmigen Kanals in Richtung der distalen Stirnfläche beträgt vorzugsweise in etwa 3% - 25%, insbesondere in etwa 5% - 15%, vorzugsweise zwischen 8 % - 9 %.

Vorzugsweise windet der zumindest eine wendelförmige Kanal sich mit einem Drehwinkel im Bereich zwischen 45° und 180° um die Längsachse des Arbeitsabschnitts. Der zumindest eine wendelförmige Kanal windet sich zum Beispiel mit in etwa einer 1/8 bis ¼-Drehung um die Längsachse des Arbeitsabschnitts.

Vorzugsweise ist die zumindest eine Öffnung der Flüssigkeitsleitung in jener Hälfte des zumindest einen, sich wendelförmig um die Längsachse des Arbeitsabschnitts erstreckenden Kanals vorgesehen, die näher zur distalen Stirnfläche angeordnet ist. Damit ist eine optimale Versorgung und Kühlung der Mantelfläche des Arbeitsabschnitts, der daran angrenzenden Knochenschicht, der distalen Stirnfläche und der damit bearbeiteten Knochenfläche erzielbar.

Wie im Vorstehenden bereits beschrieben sind an der distalen Stirnfläche, zwischen den Schneidkanten Rinnen zur Leitung von Flüssigkeit vorgesehen, die mit dem zumindest einen, sich wendelförmig um die Längsachse des Arbeitsabschnitts erstreckenden Kanal verbunden sind oder in diesen münden. Die Rinnen sind insbesondere durch von den Schneidkanten entspringende, in Richtung der Mantelfläche des Arbeitsabschnitts oder des Werkzeugkörpers geneigte Flächen gebildet. Durch die Rinnen ist eine optimale Leitung der Flüssigkeit von der zumindest einen Öffnung der Flüssigkeitsleitung durch den zumindest einen Kanal bis an die distale Stirnfläche und / oder die Behandlungsstelle und / oder eine optimale Ableitung der Flüssigkeit und insbesondere darin enthaltener Partikel, zum Beispiel durch die Schneidkanten abgetragener Knochenpartikel, von der distalen Stirnfläche und / oder der Behandlungsstelle weg (in Richtung des Werkzeugkörpers) möglich.

Vorzugsweise erstrecken sich die Rinnen zur Leitung von Flüssigkeit, insbesondere entsprechend den Schneidkanten, radial von einem Mittelpunkt der distalen Stirnfläche in Richtung des Umfangs der distalen Stirnfläche. Die distale Stirnfläche ist damit sternförmig mit von ihrem Mittelpunkt ausgehenden, abwechselnd angeordneten Rinnen und Schneidkanten ausgebildet. Vorzugsweise sind die Schneidkanten und die Rinnen zur Leitung von Flüssigkeit in Richtung einer zentral in der distalen Stirnfläche angeordneten Vertiefung geneigt. Besonders bevorzugt ist in dem Mittelpunkt oder der Vertiefung der distalen Stirnfläche eine Öffnung der Flüssigkeitsleitung vorgesehen.

Vorzugsweise ist ein an dem Ultraschallwerkzeug ausgebildeter Flüssigkeitspfad vorgesehen, der folgende, im Vorstehenden genannten Elemente umfasst: die Flüssigkeitsleitung, die zumindest eine Öffnung zum Austritt einer in der Flüssigkeitsleitung fließenden Flüssigkeit aus dem Ultraschallwerkzeug, den zumindest einen, sich wendelförmig um die Längsachse des Arbeitsabschnitts erstreckenden Kanal und die Rinnen zur Leitung von Flüssigkeit an der distalen Stirnfläche. Der Flüssigkeitspfad ist insbesondere dazu ausgebildet, eine Flüssigkeit durch diese genannten Elemente des Ultraschallwerkzeugs an die distale Stirnfläche und / oder eine Behandlungsstelle zu leiten, wodurch eine ausgezeichnete Kühlung des gesamten Arbeitsabschnitts und Verteilung der Flüssigkeit auf den zu bearbeitenden Knochen und den Arbeitsabschnitt möglich ist.

Besonders bevorzugt umfasst der Flüssigkeitspfad des Weiteren zumindest einen, sich wendelförmig um die Längsachse des Arbeitsabschnitts erstreckenden Kanal, der ausgebildet ist, Flüssigkeit von der distalen Stirnfläche und / oder der Behandlungsstelle wegzuleiten. In oder an diesem zumindest eine Kanal zum Ableiten der Flüssigkeit ist insbesondere keine Öffnung zum Austritt einer in der Flüssigkeitsleitung fließenden Flüssigkeit vorgesehen. Von der Flüssigkeit wird insbesondere auch durch die Schneidelemente abgetragenes Material mitgenommen und von der Behandlungsstelle entfernt, wodurch die Abtragungswirkung der Schneidelemente verbessert wird.

Vorzugsweise ist an zumindest einem Abschnitt des Ultraschallwerkzeugs, insbesondere an dem Arbeitsabschnitt und / oder an zumindest einem Teil des Körpers, eine Beschichtung zum Schutz vor Korrosion vorgesehen, zum Beispiel eine Titan-Nitrid-Beschichtung.

Eine dentale oder dentalchirurgische Behandlungsvorrichtung umfasst ein im Vorstehenden beschriebenes Ultraschallwerkzeug und eine Ultraschallquelle zur Erzeugung von Ultraschallschwingungen, die mit dem Ultraschallwerkzeug verbindbar ist, so dass die von der Ultraschallquelle erzeugten Ultraschallschwingungen auf das Ultraschallwerkzeug übertragbar sind. Die Ultraschallquelle ist zum Beispiel als Piezoschwinger oder als magnetostriktiver Schwinger ausgebildet. Die Ultraschallquelle ist bevorzugt in einem dentalen oder dentalchirurgischen Handstück angeordnet.

Die Behandlungsvorrichtung oder das Handstück umfassen bevorzugt zumindest ein weiteres der folgenden Bauteile: Eine Werkzeugverbindungsvorrichtung zur lösbaren Verbindung mit dem Ultraschallwerkzeug, die insbesondere den im Vorstehenden beschriebenen Gewindestift umfasst; eine elektrische oder elektronische Steuerung zum Betrieb der Behandlungsvorrichtung oder des Handstücks, insbesondere der Ultraschallquelle; einen Schwingungsüberträger oder eine Sonotrode zum Übertragen der von der Ultraschallquelle erzeugten Ultraschallschwingungen auf das Ultraschallwerkzeug; eine Beleuchtungsvorrichtung, vorzugsweise mit LEDs, insbesondere mit einer ringförmigen Lichtabgabefläche an dem im Betrieb der Behandlungsstelle zugewandten Ende, wobei die ringförmige Lichtabgabefläche das Ultraschallwerkzeug umgibt; zumindest eine Leitung zum Übertragen der Flüssigkeit von einer Flüssigkeitsquelle an das Ultraschallwerkzeug.

Die Erfindung wird nachfolgend anhand eines bevorzugten Ausführungsbeispiels und Bezug nehmend auf die beigefügten Zeichnungen erläutert. Es zeigt die
Figur 1 eine perspektivische Ansicht eines dentalen oder dentalchirurgischen Ultraschallwerkzeugs;
Figur 2 eine Schnittdarstellung durch das Ultraschallwerkzeug der Figur 1;
Figur 3 eine vergrößerte Darstellung des distalen Endabschnitts des Ultraschallwerkzeugs der Figur 1;
Figur 4 eine Vorderansicht des Arbeitsabschnitts des Ultraschallwerkzeugs der Figur 1.

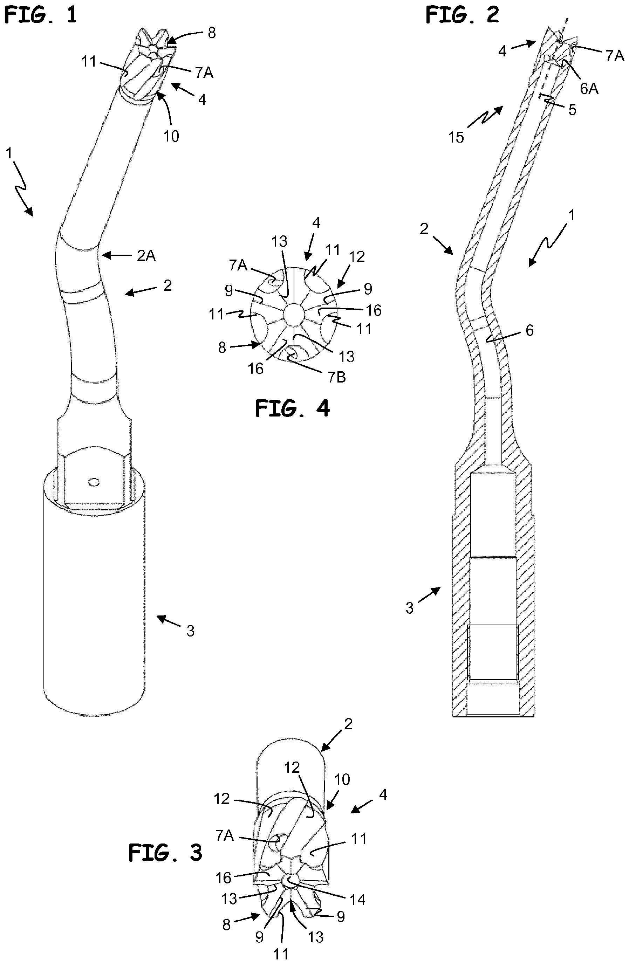

Das in den Figuren 1 - 4 dargestellte dentale oder dentalchirurgische Ultraschallwerkzeug 1 umfasst einen länglichen Werkzeugkörper 2, eine Anschlussvorrichtung 3 zum lösbaren Anschluss des Ultraschallwerkzeugs 1 an eine Ultraschallquelle, eine Arbeitsabschnitt 4, der sich entlang einer Längsachse 5 erstreckt und der ausgebildet ist, aufgrund der von der Ultraschallquelle erzeugten und über die Anschlussvorrichtung 3 und den Werkzeugkörper 2 übertragenen Ultraschallschwingungen in einen zu behandelnden Knochen einzudringen, und eine Flüssigkeitsleitung 6, die sich durch den Werkzeugkörper 2 in Richtung des Arbeitsabschnitts 4 erstreckt und in mehreren, zum Beispiel zwei, Öffnungen 7A, 7B endet, die zum Austritt einer in der Flüssigkeitsleitung 6 fließenden Flüssigkeit aus dem Ultraschallwerkzeug 1 vorgesehen sind.

Zur Erleichterung der Handhabung des Ultraschallwerkzeugs 1 weist der längliche Werkzeugkörper 2 eine Biegung 2A auf, so dass der Arbeitsabschnitt 4 oder dessen Längsachse 5 gewinkelt zur Anschlussvorrichtung 3 angeordnet sind. Der längliche Werkzeugkörper 2 umfasst des Weiteren ein erstes Ende, an dem die Anschlussvorrichtung 3 vorgesehen ist, und ein zweites Ende, an das der Arbeitsabschnitt 4 anschließt.

Der Arbeitsabschnitt 4 weist eine distale Stirnfläche 8 mit mehreren, zum Beispiel fünf, Schneidkanten 9 und eine sich von der distalen Stirnfläche 8 in Richtung des Werkzeugkörpers 2 erstreckende Mantelfläche 10 auf. In der Mantelfläche 10 sind mehrere, zum Beispiel fünf, Kanäle 11 zur Flüssigkeitsleitung vorgesehen. Die Kanäle 11 erstrecken sich jeweils von der distalen Stirnfläche 8 in Richtung des Werkzeugkörpers 2. Die Kanäle sind gleichmäßig voneinander beabstandet auf der Mantelfläche 10 verteilt.

Die Flüssigkeitsleitung 6 mündet mit ihren Öffnungen 7A, 7B an der Mantelfläche 10 des Arbeitsabschnitts 4. Die Öffnungen 7A, 7B sind in den Kanälen 11 angeordnet, wobei sich in einem Kanal 11 jeweils nur eine Öffnung 7A, 7B befindet. Wie insbesondere aus den Figuren 3 und 4 zu erkennen ist, ist nur in zwei Kanälen 11 jeweils eine Öffnungen 7A, 7B vorgesehen und die drei anderen wendelförmigen Kanäle 11 weisen keine Öffnung 7A, 7B der Flüssigkeitsleitung 6 auf. Die wendelförmigen Kanäle mit den Öffnungen 7A, 7B sind vorzugsweise durch zumindest einen wendelförmigen Kanal 11 ohne Öffnungen 7A, 7B getrennt.

Die Verbindung zwischen der im Wesentlichen zentral in dem Werkzeugkörper 2 angeordneten Flüssigkeitsleitung 6 und den Öffnungen 7A, 7B ist durch mehrere Leitungsarme 6A bewerkstelligt, wobei jede der Öffnungen 7A, 7B über einen eigenen Leitungsarm 6A mit der Flüssigkeitsleitung 6 verbunden ist. Die Leitungsarme 6A sind gewinkelt zur Flüssigkeitsleitung 6 angeordnet.

Die Kanäle 11 zur Flüssigkeitsleitung erstrecken sich wendelförmig um die Längsachse 5 des Arbeitsabschnitts 4, mit einem Drehwinkel von in etwa 70°. Die wendelförmigen Kanäle 11 sind durch ebenfalls wendelförmige Stege 12 voneinander getrennt. Die Kanäle 11 sind in Bezug auf die Stege 12 als Vertiefungen in die Mantelfläche 10 ausgebildet.

An der distalen Stirnfläche 8, zwischen den Schneidkanten 9 sind mehrere, zum Beispiel fünf, Rinnen 13 zur Leitung von Flüssigkeit vorgesehen. Die Rinnen 13 sind durch von den Schneidkanten 9 entspringende, in Richtung der Mantelfläche 10 des Arbeitsabschnitts 4 oder des Werkzeugkörpers 2 geneigte Flächen 16 gebildet. Jeweils eine Rinne 13 ist mit einem sich wendelförmig um die Längsachse 5 des Arbeitsabschnitts 4 erstreckenden Kanal 11 verbunden. Die Schneidkanten 9 und die Rinnen 13 zur Leitung von Flüssigkeit erstrecken sich radial von einem Mittelpunkt der distalen Stirnfläche 8. Die Schneidkanten 9 und die Rinnen 13 sind des Weiteren in Richtung einer zentral um den Mittelpunkt der distalen Stirnfläche 8 angeordneten Vertiefung 14 geneigt.

Die Flüssigkeitsleitung 6, die beiden Öffnungen 7A, 7B zum Austritt einer in der Flüssigkeitsleitung 6 fließenden Flüssigkeit, die sich wendelförmig um die Längsachse 5 des Arbeitsabschnitts 4 erstreckenden Kanäle 11, insbesondere jene Kanäle 11, in oder an denen die Öffnungen 7A, 7B angeordnet sind, und die Rinnen 13 an der distalen Stirnfläche 8 bilden zusammen einen Flüssigkeitspfad 15, um eine Flüssigkeit von der Flüssigkeitsleitung 6 bis an die distale Stirnfläche 8 und / oder eine Behandlungsstelle zu leiten und den Arbeitsabschnitt 4 und die Behandlungsstelle zu kühlen. Insbesondere jene wendelförmigen Kanäle 11, die keine Öffnungen 7A, 7B aufweisen und die Teil des Flüssigkeitspfads 15 sind, sind dazu vorgesehen, Flüssigkeit von der distalen Stirnfläche 8 und / oder der Behandlungsstelle in Richtung des Werkzeugkörpers 2 wegzuleiten.

## Patentansprüche

1. Dentales oder dentalchirurgisches Ultraschallwerkzeug (1), umfassend: einen länglichen Werkzeugkörper (2) mit einer Biegung (2A), eine Anschlussvorrichtung (3) zum lösbaren Anschluss des Ultraschallwerkzeugs (1) an eine Ultraschallquelle, wobei die Anschlussvorrichtung (3) an einem ersten Ende des Werkzeugkörpers (2) vorgesehen ist, einen an einem zweiten Ende des Werkzeugkörpers (2) vorgesehenen Arbeitsabschnitt (4), der sich entlang einer Längsachse (5) erstreckt und der ausgebildet ist, aufgrund der von der Ultraschallquelle erzeugten und über die Anschlussvorrichtung (3) und den Werkzeugkörper (2) übertragenen Ultraschallschwingungen in einen zu behandelnden Knochen einzudringen, und eine Flüssigkeitsleitung (6), die sich durch den Werkzeugkörper (2) in Richtung des Arbeitsabschnitts (4) erstreckt und in zumindest einer Öffnung (7A, 7B) endet, die zum Austritt einer in der Flüssigkeitsleitung (6) fließenden Flüssigkeit aus dem Ultraschallwerkzeug (1) vorgesehen ist, wobei der Arbeitsabschnitt (4) eine distale Stirnfläche (8) mit mehreren Schneidkanten (9) und eine sich von der distalen Stirnfläche (8) in Richtung des Werkzeugkörpers (2) erstreckende Mantelfläche (10) aufweist, wobei in der Mantelfläche (10) zumindest ein Kanal (11) zur Flüssigkeitsleitung vorgesehen ist, der sich von der distalen Stirnfläche (8) in Richtung des Werkzeugkörpers (2) erstreckt, **dadurch gekennzeichnet, dass** der zumindest eine Kanal (11) zur Flüssigkeitsleitung sich wendelförmig um die Längsachse (5) des Arbeitsabschnitts (4) erstreckt.

2. Dentales oder dentalchirurgisches Ultraschallwerkzeug (1) nach Anspruch 1, **dadurch gekennzeichnet, dass**
die zumindest eine Öffnung (7A, 7B) der Flüssigkeitsleitung (6) an der Mantelfläche (10) des Arbeitsabschnitts (4) in dem zumindest einen sich wendelförmig um die Längsachse (5) des Arbeitsabschnitts (4) erstreckenden Kanal (11) zur Flüssigkeitsleitung oder unmittelbar anschließend an den zumindest einen wendelförmigen Kanal (11) zur Flüssigkeitsleitung angeordnet ist.

3. Dentales oder dentalchirurgisches Ultraschallwerkzeug (1) nach Anspruch 1 oder 2, **gekennzeichnet durch**
mehrere, sich wendelförmig um die Längsachse (5) des Arbeitsabschnitts (4) erstreckende Kanäle (11), die durch, insbesondere wendelförmige, Stege (12) voneinander getrennt sind.

4. Dentales oder dentalchirurgisches Ultraschallwerkzeug (1) nach einem der Ansprüche 1 - 3, **gekennzeichnet durch**
mehrere, sich wendelförmig um die Längsachse (5) des Arbeitsabschnitts (4) erstreckende Kanäle (11) und mehrere Öffnungen (7A, 7B) der Flüssigkeitsleitung (6), wobei in oder an einem Kanal (11) jeweils nur eine Öffnung (7A, 7B) angeordnet ist.

5. Dentales oder dentalchirurgisches Ultraschallwerkzeug (1) nach einem der vorstehenden Ansprüche, **dadurch gekennzeichnet, dass**
zumindest in oder an einem, sich wendelförmig um die Längsachse (5) des Arbeitsabschnitts (4) erstreckenden Kanal (11) keine Öffnung (7A, 7B) der Flüssigkeitsleitung (6) vorgesehen ist.

6. Dentales oder dentalchirurgisches Ultraschallwerkzeug (1) nach einem der vorstehenden Ansprüche, **dadurch gekennzeichnet, dass**
der zumindest eine, sich wendelförmig um die Längsachse (5) des Arbeitsabschnitts (4) erstreckende Kanal (11) eine in Richtung der distalen Stirnfläche (8) zunehmende Tiefe aufweist.

7. Dentales oder dentalchirurgisches Ultraschallwerkzeug (1) nach einem der vorstehenden Ansprüche, **dadurch gekennzeichnet, dass**
die Steigung des zumindest einen sich wendelförmig um die Längsachse (5) des Arbeitsabschnitts (4) erstreckende Kanals (11) etwa 5 mm - 50 mm beträgt.

8. Dentales oder dentalchirurgisches Ultraschallwerkzeug (1) nach einem der vorstehenden Ansprüche, **dadurch gekennzeichnet, dass**
der zumindest eine sich wendelförmig um die Längsachse (5) des Arbeitsabschnitts (4) erstreckende Kanal (11) sich mit einem Drehwinkel im Bereich zwischen 45° und 180° um die Längsachse (5) windet.

9. Dentales oder dentalchirurgisches Ultraschallwerkzeug (1) nach einem der Ansprüche 2 - 8, **dadurch gekennzeichnet, dass**
die zumindest eine Öffnung (7A, 7B) der Flüssigkeitsleitung (6) in jener Hälfte des zumindest einen, sich wendelförmig um die Längsachse (5) des Arbeitsabschnitts (4) erstreckenden Kanals (11) vorgesehen ist, die näher zur distalen Stirnfläche (8) angeordnet ist.

10. Dentales oder dentalchirurgisches Ultraschallwerkzeug (1) nach einem der Ansprüche 1 - 9, **dadurch gekennzeichnet, dass**
an der distalen Stirnfläche (8), zwischen den Schneidkanten (9) Rinnen (13) zur Leitung von Flüssigkeit vorgesehen sind, die mit dem zumindest einen, sich wendelförmig um die Längsachse (5) des Arbeitsabschnitts (4) erstreckenden Kanal (11) verbunden sind oder in diesen münden.

11. Dentales oder dentalchirurgisches Ultraschallwerkzeug (1) nach Anspruch 10, **dadurch gekennzeichnet, dass**
die Schneidkanten (9) und die Rinnen (13) zur Leitung von Flüssigkeit sich radial von einem Mittelpunkt der distalen Stirnfläche (8) erstrecken.

12. Dentales oder dentalchirurgisches Ultraschallwerkzeug (1) nach Anspruch 10 oder 11, **dadurch gekennzeichnet, dass**
die Schneidkanten (9) und die Rinnen (13) zur Leitung von Flüssigkeit in Richtung einer zentral in der distalen Stirnfläche (8) angeordneten Vertiefung (14) geneigt sind.

13. Dentales oder dentalchirurgisches Ultraschallwerkzeug (1) nach einem der vorstehenden Ansprüche, **gekennzeichnet durch**
einen an dem Ultraschallwerkzeug (1) ausgebildeten Flüssigkeitspfad (15), der die Flüssigkeitsleitung (6), die zumindest eine Öffnung (7A, 7B) zum Austritt einer in der Flüssigkeitsleitung (6) fließenden Flüssigkeit aus dem Ultraschallwerkzeug (1), den zumindest einen, sich wendelförmig um die Längsachse (5) des Arbeitsabschnitts (4) erstreckenden Kanal (11) und die Rinnen (13) zur Leitung von Flüssigkeit an der distalen Stirnfläche (8) umfasst, um eine Flüssigkeit durch die Flüssigkeitsleitung (6), die zumindest eine Öffnung (7A, 7B), den zumindest einen, sich wendelförmig um die Längsachse (5) des Arbeitsabschnitts (4) erstreckenden Kanal (11) und die Rinnen (13) zur Leitung von Flüssigkeit an die distale Stirnfläche (8) und / oder eine Behandlungsstelle zu leiten.

14. Dentales oder dentalchirurgisches Ultraschallwerkzeug (1) nach Anspruch 13, **dadurch gekennzeichnet, dass**
der Flüssigkeitspfad (15) des Weiteren einen, sich wendelförmig um die Längsachse (5) des Arbeitsabschnitts (4) erstreckenden, Kanal (11) aufweist, der ausgebildet ist, Flüssigkeit von der distalen Stirnfläche (8) und / oder der Behandlungsstelle wegzuleiten.

15. Dentale oder dentalchirurgische Behandlungsvorrichtung, **gekennzeichnet durch** ein Ultraschallwerkzeug (1) gemäß zumindest einem der vorstehenden Ansprüche und eine Ultraschallquelle zur Erzeugung von Ultraschallschwingungen, die mit dem Ultraschallwerkzeug (1) verbindbar ist, so dass die von der Ultraschallquelle erzeugten Ultraschallschwingungen auf das Ultraschallwerkzeug (1) übertragbar sind.

## Claims

1. A dental or dental surgical ultrasonic tool (1), comprising: an elongated tool body (2) having a bend (2A), a connecting device (3) for releasable connection of the ultrasonic tool (1) to an ultrasonic source, wherein the connecting device (3) is provided on a first end of the tool body (2), a working section (4) which is provided on a second end of the tool body (2) and extends along a longitudinal axis (5) and is configured to penetrate into bone to be treated based on ultrasonic vibrations generated by the ultrasonic source and transmitted via the connecting device (3) and the tool body (2), and a liquid line (6) extending through the tool body (2) in the direction of the working section (4) and ending in at least one opening (7A, 7B), which is provided for the discharge of a liquid flowing in the liquid line (6) out of the ultrasonic tool (1), wherein the working section (4) comprises a distal end face (8) with a plurality of cutting edges (9) and a lateral surface (10) extending from the distal end face (8) in the direction of the tool body (2), wherein at least one channel (11) for conveying a liquid is provided in the lateral surface (10), extending from the distal end face (8) in the direction of the tool body (2), **characterized in that** the at least one channel (11) for conveying a liquid extends in a helical form around the longitudinal axis (5) of the working section (4).

2. The dental or dental surgical ultrasonic tool (1) according to claim 1, **characterized in that**
the at least one opening (7A, 7B) of the liquid line (6) is disposed on the lateral surface (10) of the working section (4) in the at least one channel (11) for conveying a liquid which extends in a helical form around the longitudinal axis (5) of the working section (4) or directly adjacent to the at least one helical channel (11) for conveying a liquid.

3. The dental or dental surgical ultrasonic tool (1) according claim 1 or 2, **characterized by**
a plurality of channels (11) extending in a helical form around the longitudinal axis (5) of the working section (4) and which are separated from one another by webs (12), having in particular a helical form.

4. The dental or dental surgical ultrasonic tool (1) according to any one of claims 1-3, **characterized by**
a plurality of channels (11) extending in a helical form around the longitudinal axis (5) of the working section (4) and a plurality of openings (7A, 7B) of the liquid line (6), wherein only one opening (7A, 7B) is disposed in or at each channel (11).

5. The dental or dental surgical ultrasonic tool (1) according to any one of the preceding claims, **characterized in that**
no opening (7A, 7B) of the liquid line (6) is provided at least in or at one channel (11) extending in a helical form around the longitudinal axis (5) of the working section.

6. The dental or dental surgical ultrasonic tool (1) according to any one of the preceding claims, **characterized in that**
the at least one channel (11) extending in a helical form around the longitudinal axis (5) of the working section (4) has a depth that increases in the direction of the distal end face (8).

7. The dental or dental surgical ultrasonic tool (1) according to any one of the preceding claims, **characterized in that**
a slope of the at least one channel (11) extending in a helical form around the longitudinal axis (5) of the working section (4) amounts to approx. 5 mm - 50 mm.

8. The dental or dental surgical ultrasonic tool (1) according to any one of the preceding claims, **characterized in that**
the at least one channel (11) extending in a helical form around the longitudinal axis (5) of the working section (4) winds at a rotational angle in the range between 45° and 180° around the longitudinal axis (5).

9. The dental or dental surgical ultrasonic tool (1) according to any one of claims 2 - 8, **characterized in that**
the at least one opening (7A, 7B) of the liquid line (6) is provided in this half of the at least one channel (11) extending in a helical form around the longitudinal axis (5) of the working section (4) which is disposed closer to the distal end face (8).

10. The dental or dental surgical ultrasonic tool (1) according to any one of claims 1 - 9, **characterized in that**
troughs (13) for carrying liquid are provided on the distal end face (8) between the cutting edges (9) and connect to or open into the at least one channel (11) extending in a helical form around the longitudinal axis (5) of the working section (4).

11. The dental or dental surgical ultrasonic tool (1) according to claim 10, **characterized in that**
the cutting edges (9) and the troughs (13) for carrying liquid extend radially out from a midpoint on the distal end face (8).

12. The dental or dental surgical ultrasonic tool (1) according to claim 10 or 11, **characterized in that**
the cutting edges (9) and the troughs (13) for carrying liquid are inclined in the direction of a recess (14) disposed centrally in the distal end face (8).

13. The dental or dental surgical ultrasonic tool (1) according to any one of the preceding claims, **characterized by**
a liquid path (15) formed on the ultrasonic tool (1), comprising the liquid line (6), the at least one opening (7A, 7B) for the discharge of a liquid flowing in the liquid line (6) out of the ultrasonic tool (1), the at least one channel (11) extending in a helical form around the longitudinal axis (5) of the working section (4), and the troughs (13) for carrying liquid on the distal end face (8), in order to carry a liquid through the liquid line (6), the at least one opening (7A, 7B), the at least one channel (11) extending in a helical form around the longitudinal axis (5) of the working section (4), and the troughs (13) for carrying liquid to the distal end face (8) and/or a treatment site.

14. The dental or dental surgical ultrasonic tool (1) according to claim 13, **characterized in that**
the liquid path (15) in addition comprises a channel (11) extending in a helical form around the longitudinal axis (5) of the working section (4) and which is configured to carry liquid away from the distal end face (8) and/or the treatment site.

15. A dental or dental surgical treatment device, **characterized by** an ultrasonic tool (1) according to at least one of the preceding claims and an ultrasonic source for generating ultrasonic vibrations which can be connected to the ultrasonic tool (1) so that the ultrasonic vibrations generated by the ultrasonic source can be transmitted to the ultrasonic tool (1).

## Revendications

1. Outil ultrasonique (1) dentaire ou de chirurgie dentaire, comprenant: un corps d'outil oblong (2) avec une courbure (2A), un dispositif de raccordement (3) pour le raccordement détachable de l'outil ultrasonique (1) à une source d'ultrasons, dans lequel le dispositif de raccordement (3) est prévu à une première extrémité du corps d'outil (2), un segment de travail (4) prévu à une deuxième extrémité du corps d'outil (2), lequel s'étend le long d'un axe longitudinal (5) et est réalisé, en raison des vibrations ultrasonores générées par la source d'ultrasons et transmises via le dispositif de raccordement (3) et le corps d'outil (2), pour pénétrer dans un os à traiter, et une conduite de liquide (6) qui s'étend à travers le corps d'outil (2) en direction du segment de travail (4) et se termine dans au moins une ouverture (7A, 7B), laquelle est prévue pour la sortie d'un liquide circulant dans la conduite de liquide (6) hors de l'outil ultrasonique (1), dans lequel le segment de travail (4) présente une surface frontale distale (8) avec plusieurs arêtes de coupe (9) et une surface enveloppante (10) s'étendant de la surface frontale distale (8) en direction du corps d'outil (2), dans lequel au moins un canal (11) est prévu dans la surface enveloppante (10) pour le guidage de liquide, lequel canal (11) s'étend de la surface frontale distale (8) en direction du corps d'outil (2), **caractérisé en ce que** l'au moins un canal (11) pour le guidage de liquide s'étend hélicoïdalement autour de l'axe longitudinal (5) du segment de travail (4).

2. Outil ultrasonique (1) dentaire ou de chirurgie dentaire selon la revendication 1, **caractérisé en ce que**
l'au moins une ouverture (7A, 7B) de la conduite de liquide (6) est disposée au niveau de la surface enveloppante (10) du segment de travail (4) dans l'au moins un canal (11) s'étendant hélicoïdalement autour de l'axe longitudinal (5) du segment de travail (4) pour le guidage du liquide, ou de manière directement adjacente à l'au moins un canal hélicoïdal (11) pour le guidage de liquide.

3. Outil ultrasonique (1) dentaire ou de chirurgie dentaire selon la revendication 1 ou 2, **caractérisé par**
plusieurs canaux (11) s'étendant hélicoïdalement autour de l'axe longitudinal (5) du segment de travail (4), lesquels sont séparés les uns des autres par des nervures (12) en particulier hélicoïdales.

4. Outil ultrasonique (1) dentaire ou de chirurgie dentaire selon l'une des revendications 1-3, **caractérisé par**
plusieurs canaux (11) s'étendant hélicoïdalement autour de l'axe longitudinal (5) du segment de travail (4) et plusieurs ouvertures (7A, 7B) de la conduite de liquide (6), dans lequel respectivement seulement une ouverture (7A, 7B) est disposée dans un canal (11) ou au niveau de celui-ci.

5. Outil ultrasonique (1) dentaire ou de chirurgie dentaire selon l'une des revendications précédentes, **caractérisé en ce que**,
au moins dans un canal (11), ou au niveau de celui-ci, s'étendant hélicoïdalement autour de l'axe longitudinal (5) du segment de travail (4), aucune ouverture (7A, 7B) de la conduite de liquide (6) n'est prévue.

6. Outil ultrasonique (1) dentaire ou de chirurgie dentaire selon l'une des revendications précédentes, **caractérisé en ce que**
l'au moins un canal (11) s'étendant hélicoïdalement autour de l'axe longitudinal (5) du segment de travail (4) présente une profondeur augmentant en direction de la surface frontale distale (8).

7. Outil ultrasonique (1) dentaire ou de chirurgie dentaire selon l'une des revendications précédentes, **caractérisé en ce que**
le pas de l'au moins un canal (11) s'étendant hélicoïdalement autour de l'axe longitudinal (5) du segment de travail (4) comporte environ 5 mm - 50 mm.

8. Outil ultrasonique (1) dentaire ou de chirurgie dentaire selon l'une des revendications précédentes, **caractérisé en ce que**
l'au moins un canal (11) s'étendant hélicoïdalement autour de l'axe longitudinal (5) du segment de travail (4) s'enroule avec un angle de rotation dans la plage comprise entre 45° et 180° autour de l'axe longitudinal (5).

9. Outil ultrasonique (1) dentaire ou de chirurgie dentaire selon l'une des revendications 2 - 8, **caractérisé en ce que**
l'au moins une ouverture (7A, 7B) de la conduite de liquide (6) est prévue dans cette moitié de l'au moins un canal (11) s'étendant hélicoïdalement autour de l'axe longitudinal (5) du segment de travail (4) qui est disposée plus près de la surface frontale distale (8).

10. Outil ultrasonique (1) dentaire ou de chirurgie dentaire selon l'une quelconque des revendications 1 - 9, **caractérisé en ce que**,
au niveau de la surface frontale distale (8), entre les arêtes de coupe (9), des gorges (13) sont prévues pour le guidage de liquide, lesquelles sont en liaison avec l'au moins un canal (11) s'étendant hélicoïdalement autour de l'axe longitudinal (5) du segment de travail (4), ou débouchent dans celui-ci.

11. Outil ultrasonique (1) dentaire ou de chirurgie dentaire selon la revendication 10, **caractérisé en ce que**
les arêtes de coupe (9) et les gorges (13) pour le guidage de liquide s'étendent radialement d'un point médian de la surface frontale distale (8).

12. Outil ultrasonique (1) dentaire ou de chirurgie dentaire selon la revendication 10 ou 11, **caractérisé en ce que**
les arêtes de coupe (9) et les gorges (13) pour le guidage de liquide sont inclinées en direction d'un creux (14) disposé centralement dans la surface frontale distale (8).

13. Outil ultrasonique (1) dentaire ou de chirurgie dentaire selon l'une des revendications précédentes, **caractérisé par**
un trajet de liquide (15) formé au niveau de l'outil ultrasonique (1), lequel comprend la conduite de liquide (6), l'au moins une ouverture (7A, 7B) pour la sortie d'un liquide circulant dans la conduite de liquide (6) hors de l'outil ultrasonique (1), l'au moins un canal (11) s'étendant hélicoïdalement autour de l'axe longitudinal (5) du segment de travail (4), et les gorges (13) pour le guidage de liquide au niveau de la surface frontale distale (8), pour guider un liquide à travers la conduite de liquide (6), l'au moins une ouverture (7A, 7B), l'au moins un canal (11) s'étendant hélicoïdalement autour de l'axe longitudinal (5) du segment de travail (4) et les gorges (13) pour le guidage de liquide vers la surface frontale distale (8) et/ou un emplacement de traitement.

14. Outil ultrasonique (1) dentaire ou de chirurgie dentaire selon la revendication 13, **caractérisé en ce que**
le trajet de liquide (15) présente en outre un canal (11) s'étendant hélicoïdalement autour de l'axe longitudinal (5) du segment de travail (4), lequel est réalisé pour conduire du liquide loin de la surface frontale distale (8) et/ou de l'emplacement de traitement.

15. Dispositif de traitement dentaire ou de chirurgie dentaire, **caractérisé par** un outil ultrasonique (1) selon l'une au moins des revendications précédentes et une source d'ultrasons pour la génération de vibrations ultrasonores, laquelle peut être raccordée à l'outil ultrasonique (1), de sorte que les vibrations ultrasonores générées par la source d'ultrasons peuvent être transmises à l'outil ultrasonique (1).
